(19) Europäisches Patentamt
European Patent Office
Office européen des brevets

(11) Veröffentlichungsnummer: **0 435 220 A2**

(12) # EUROPÄISCHE PATENTANMELDUNG

(21) Anmeldenummer: **90125271.8**

(22) Anmeldetag: **21.12.90**

(51) Int. Cl.5: **C07C 27/28**, C07C 275/14, C07D 251/32, C07C 271/20, D06M 13/425, D06M 13/432

(30) Priorität: **28.12.89 DE 3943127**

(43) Veröffentlichungstag der Anmeldung: **03.07.91 Patentblatt 91/27**

(84) Benannte Vertragsstaaten: **AT BE CH DE ES FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Knaup, Wolfgang, Dr.**
**Kanalstrasse 3**
**W-8269 Burgkirchen(DE)**
Erfinder: **Kupfer, Rainer, Dr.**
**Römerstrasse 2**
**W-8261 Kastl(DE)**
Erfinder: **Kleber, Rolf, Dr.**
**Am Trieb 41**
**W-6078 Neu-Isenburg(DE)**
Erfinder: **Jaeckel, Lothar**
**Kettelerstrasse 88**
**W-6093 Flörsheim(DE)**
Erfinder: **Gohlke, Fritz-Joachim, Dr.**
**Hochstauffenstrasse 9**
**W-8269 Burgkirchen(DE)**

(54) **Urethane aus aliphatischen Fluoralkoholen, Isocyanaten und Carbonsäuren, Verfahren zu ihrer Herstellung und ihre Verwendung.**

(57) Die beschriebenen Urethane sind aufgebaut aus aliphatischem Fluoralkohol, Triisocyanat und einer Carbonsäure. Sie werden hergestellt durch Reaktion des aliphatischen Fluoralkohols mit dem Triisocyanat zum Fluoralkohol-Triisocyanat-Addukt und durch Reaktion dieses Adduktes mit der Carbonsäure. Die neuen Urethane werden vorzugsweise zur Ausrüstung von Textilien in der Wasser-, öl- und Schmutzabweisung verwendet.

EP 0 435 220 A2

# URETHANE AUS ALIPHATISCHEN FLUORALKOHOLEN, ISOCYANATEN UND CARBONSÄUREN, VERFAHREN ZU IHRER HERSTELLUNG UND IHRE VERWENDUNG

Die Erfindung betrifft Urethane aus aliphatischen Fluoralkoholen, Isocyanaten und Carbonsäuren. Die Erfindung betrifft ferner ein Verfahren zur Herstellung dieser Urethane und ihre Verwendung.

Es ist schon seit langem bekannt, Urethane aus aliphatischen Fluoralkoholen und Mono- oder Polyisocyanaten durch Inkorporierung einer modifizierenden Gruppe in den Eigenschaften bezüglich Ausrüstung von Textilien, Leder, Holz und dergleichen zu verbessern. Im Laufe der Zeit sind zahlreiche Verbindungen als modifizierende Komponenten beschrieben worden, unter anderen auch Carbonsäuren.

So werden in der US-Patentschrift 4 525 305 Urethane beschrieben, die aus einem aliphatischen Fluoralkohol, der auch eine Epichlorhydringruppe enthalten kann, einem Diisocyanat und einer aliphatischen Amino- oder Hydroxy-Monocarbonsäure als modifizierende Komponente gebildet sind (vergleiche insbesondere Spalte 6, Schema 1 und Schema 2, und die Urethanverbindungen in Tabelle 1).
Ähnliche Urethanverbindungen werden ferner in der US-Patentschrift 4 539 006 beschrieben (vergleiche insbesondere Spalte 4, Zeilen 42/43, und Schema 4, Spalten 7 und 8). Die aus den beiden US-Patentschriften 4 525 305 und 4 539 006 bekannten Urethane, die insbesondere zur Ausrüstung von Leder geeignet sein sollen, weisen neben der genannten aliphatischen Monocarbonsäure offensichtlich noch die folgenden beiden charakteristischen Merkmale auf: Sie enthalten durchwegs eine Sulfonamidogruppe und als Isocyanat ausschließlich Diisocyanate.
In der neueren US-Patentschrift 4 782 175 werden Urethane aus einem aliphatischen Fluoralkohol, der bis zu 10 Epichlorhydrin-Einheiten enthält, einem Diisocyanat oder einem Triisocyanat und einer aromatischen Amino- oder Hydroxy-Monocarbonsäure beschrieben (vergleiche insbesondere Spalte 3, Zeilen 31 bis 34, und die Formel B12 in der Tabelle). Diese Urethane werden als Ausrüstungsmittel für Textilien und Leder empfohlen.

Aus dem Stand der Technik ergibt sich also, daß aliphatische und aromatische Monocarbonsäuren schon mehrfach als modifizierende Komponenten zur Herstellung von Urethanverbindungen eingesetzt worden sind und daß derartige Urethane mehr oder weniger gute Ausrüstungsmittel für Textilien und Leder sein sollen.

Der Erfindung liegt die Aufgabe zugrunde, Urethane aus aliphatischen Fluoralkoholen, Isocyanaten und Carbonsäuren zu schaffen, die im Vergleich zum Stand der Technik verbesserte Ausrüstungsmittel, insbesondere für Textilien, darstellen.

Die erfindungsgemäßen Urethane beruhen auf der überraschenden Feststellung, daß ausgewählte aliphatische, aromatische und cycloaliphatische Mono- und Polycarbonsäuren dann besonders wirksame modifizierende Komponenten sind, wenn sie mit einem aliphatischen Fluoralkohol und mit einem Triisocyanat kombiniert sind, und daß eine noch höhere Wirksamkeit erreicht wird, wenn neben einer oder mehreren freien -COOM-Gruppen (M = H oder ein Basenrest) und dem Triisocyanat auch noch Epichlorhydringruppen im Urethanmolekül vorliegen. Die erfindungsgemäßen Urethane verleihen vor allem Textilmaterialien sehr hohe Oleophobie- und Hydrophobie-Werte. Dies ist ein unerwartetes Ergebnis, zumal in den genannten US-Patentschriften 4 525 305 und 4 539 006 wiederholt darauf hingewiesen wird, daß die beschriebenen Urethane aus Fluoralkohol, aliphatischer Carbonsäure und Diisocyanat vor allem bei Leder wirksam sein sollen. Die erfindungsgemäßen Urethanverbindungen besitzen überraschenderweise noch eine weitere, sehr wertvolle Eigenschaft, nämlich die Eigenschaft, auch festen Schmutz, wie Staub (Straßenstaub), Ruß und dergleichen, abzuweisen.

Die erfindungsgemäßen Urethanverbindungen sind gekennzeichnet durch die nachstehende Formel 1

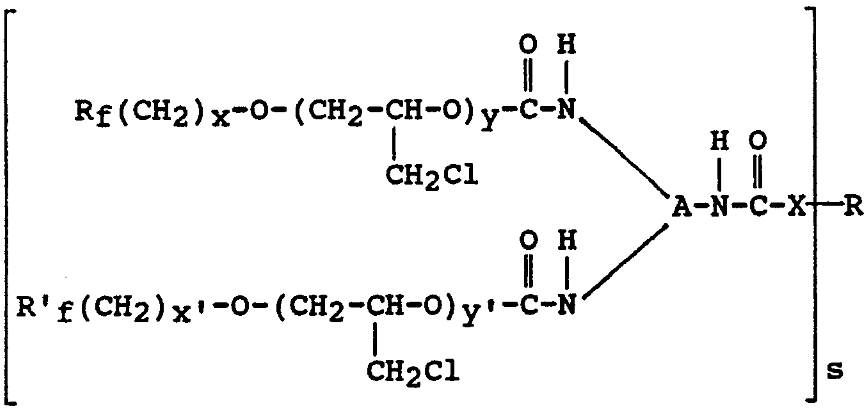 (1)

worin bedeuten:

$R_f$ und $R'_f$ jeweils eine Perfluoralkylgruppe mit 4 bis 22 C-Atomen, vorzugsweise 6 bis 18 C-Atomen; $R_f$ und $R'_f$ haben vorzugsweise die gleiche Bedeutung,

x und x' jeweils eine Zahl von 1 bis 4, vorzugsweise 2; x und x' haben vorzugsweise die gleiche Bedeutung,

y und y' jeweils eine Zahl von 0 bis 7, vorzugsweise 0 bis 4; bevorzugte Bedeutungen von y und y' sind, daß beide Indices jeweils eine Zahl von 1 bis 7 sind, vorzugsweise jeweils 1 bis 4, oder daß einer der beiden Indices 0 ist und der andere eine Zahl von 1 bis 7 ist, vorzugsweise 1 bis 4,

A einen Rest eines aliphatischen, aromatischen oder cycloaliphatischen Triisocyanates,

R einen Rest einer aromatischen, aliphatischen oder cycloaliphatischen Carbonsäure oder Carbonsäuresalzes, der 1 bis 5 -COOM-Gruppen aufweist, worin M Wasserstoff oder ein Basenrest ist,

X -O-, eine Einfachbindung (direkte Bindung) oder -NR'-,

worin R' H, $C_1$ bis $C_4$-Alkyl oder $-CH_2COOM$ ist, worin M die genannte Bedeutung hat, und

s eine Zahl von 1 bis 3, mit der Maßgabe, daß diejenigen Urethanverbindungen ausgenommen sind, wenn $R_f$ und $R'_f$ jeweils $(C_8F_{17}\text{-}C_{16}F_{33})$-, x und x' und y und y' jeweils 2 sind, A ein Triisocyanatrest der nachstehenden Formel ist,

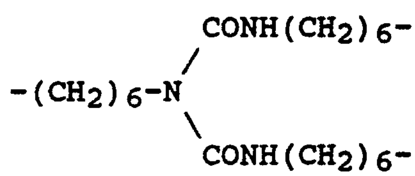

X -O- oder -NH- ist, R ein aromatischer Carbonsäurerest der nachstehenden Formel

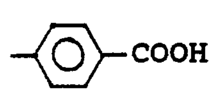

und s 1 ist.

Die ausgenommenen Urethanverbindungen ergeben sich aus der genannten US-Patentschrift 4 782 175 (vergleiche Patentanspruch 1 und Formel B12 in der Tabelle).

Die Perfluoralkylgruppe $R_f$ und $R'_f$ kann gerade oder verzweigt, gesättigt oder ungesättigt (mit vorzugsweise 1 bis 3 Doppelbindungen) sein, wobei gesättigt bevorzugt ist. Im Falle einer verzweigten Perfluoralkylgruppe ist die endverzweigte bevorzugt. Beim Perfluoralkylrest handelt es sich häufig um ein Gemisch von Perfluoralkyl mit der genannten Anzahl von C-Atomen.

A ist der Rest eines Triisocyanates. Dies ist ein wesentliches Merkmal der erfindungsgemäßen Urethane. Die Art und Struktur des Triisocyanates (das den Rest A bildet) kann in weiten Grenzen variieren. So sind aliphatische, aromatische und cycloaliphatische Triisocyanate geeignet, vorzugsweise aliphatische und aromatische. Die in Rede stehenden Triisocyanate sind bekannt und im Handel erhältlich. Nachstehend

sind Beispiele für Vertreter von A in Form der Triisocyanate formelmäßig angegeben, das sind die Formeln 2 bis 7:

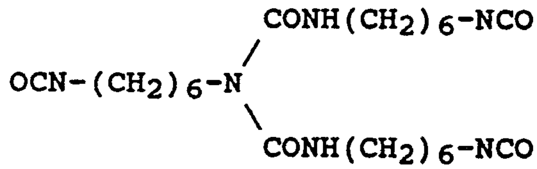

(2)

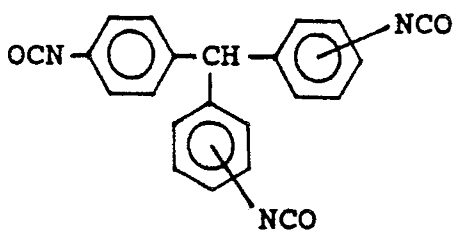

(3)

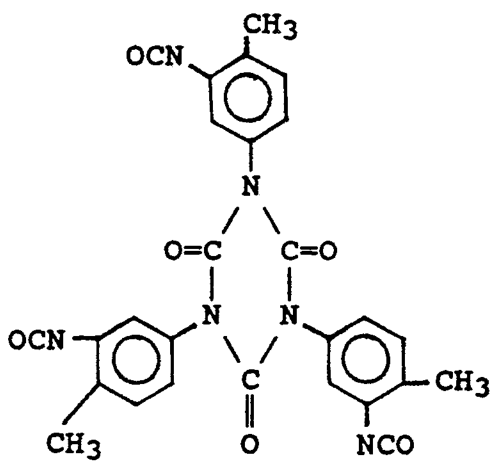

(4)

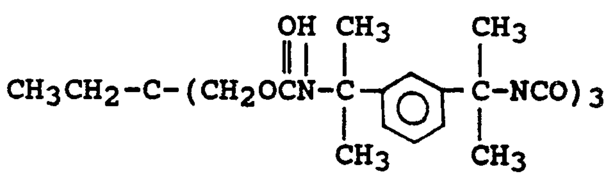

(5)

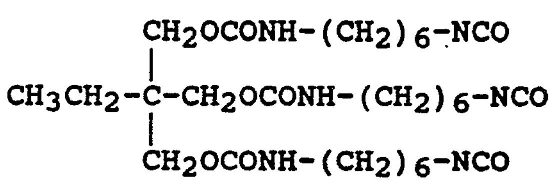

(6)

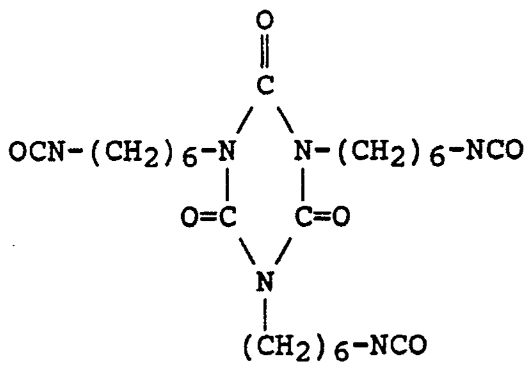

(7)

Die Triisocyanatreste, die sich aus den Formeln 2, 6 und 7 ergeben, sind besonders bevorzugte Vertreter für A.

R ist vorzugsweise (a) ein Rest einer aromatischen Carbonsäure oder eines aromatischen Carbonsäuresalzes der nachstehenden Formeln 8 und 9:

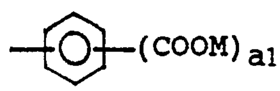

(8)

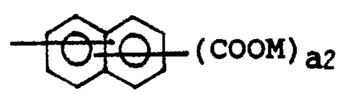

(9)

worin M die angegebene Bedeutung hat und a1 und a2 jeweils eine Zahl von 1 bis 5 sind, vorzugsweise 1, 2 oder 3, oder (b) ein Rest einer aliphatischen Carbonsäure oder eines aliphatischen Carbonsäuresalzes der nachstehenden Formel 10

$$-R^1-COOM \qquad (10)$$

worin M die angegebene Bedeutung hat und $R^1$ ein (gesättigter oder ungesättigter, substituierter oder unsubstituierter) aliphatischer Kohlenwasserstoffrest mit maximal 15 C-Atomen ist; $R^1$ bedeutet vorzugsweise einen (geraden oder ungeraden, gesättigten oder ungesättigten) Alkylenrest mit maximal 10 C-Atomen, wobei $-(CH_2)_z$-Reste, z ist eine ganze Zahl von 1 bis 10, bevorzugt sind, oder einen Rest der nachstehenden Formeln 11 bis 13:

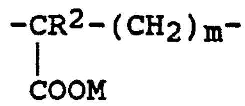

(11)

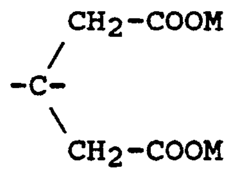

(12)

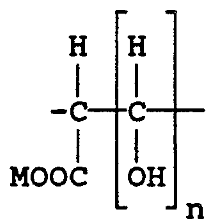

(13)

worin M die angegebene Bedeutung hat, m 0 oder eine ganze Zahl von 1 bis 7 ist, n 1, 2 oder 3 ist und $R^2$ H, OH oder $C_1$ bis $C_4$-Alkyl ist.

Nachstehend sind Beispiele für Vertreter von R in Form der Säure angegeben, das sind die Formeln 14 bis 26:

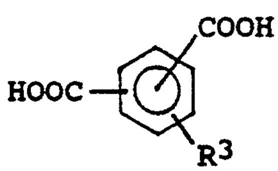

(14)

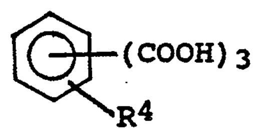

(15)

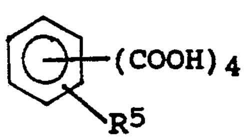

(16)

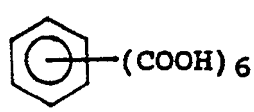

(17)

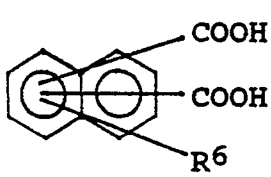

(18)

worin $R^3$, $R^4$, $R^5$, $R^6$ = H, OH oder NH2 ist,

$$HO-(CH_2)_z-COOH \quad (19)$$

das sind Hydroxyalkylencarbonsäuren,

$$H_2N-(CH_2)_z-COOH \qquad (20)$$

das sind Aminoalkylencarbonsäuren,

$$HOOC-(CH_2)_z-COOH \qquad (21)$$

das sind Alkylendicarbonsäuren,
worin z jeweils die angegebene Bedeutung hat, vorzugsweise 1 bis 5 ist,

$$HOOC-CH=CH-COOH \qquad (22)$$

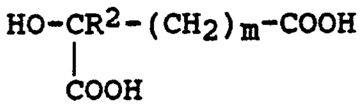

(23)

worin $R^2$ und m die angegebene Bedeutung haben, wobei für m 0, 1, 2 oder 3 und für $R^2$ H bevorzugt ist, das sind Hydroxyalkylendicarbonsäuren, wie Tartronsäure ($R^2$ = H, m = 0), Äpfelsäure ($R^2$ = H, m = 1) und dergleichen,

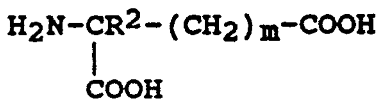

(24)

worin $R^2$ und m die bei Formel 23 angegebene Bedeutung haben, das sind Aminoalkylendicarbonsäuren,

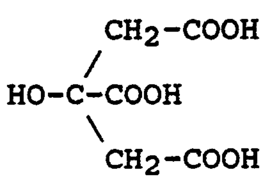

(25)

**Citronensäure**

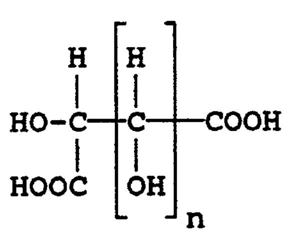

(26)

n = 1, 2, 3

**Zuckersäuren, zum Beispiel Weinsäure (n = 1)**

Besonders bevorzugte Carbonsäure- oder Carbonsäuresalzreste R sind solche, die aus den Säuren der Formeln 14 bis 16, 19 bis 21, 23 und 25 resultieren.

x bedeutet vorzugsweise -O- (das heißt eine HO-Gruppe ist der Lieferant des aktiven H-Atoms zur

Reaktion mit der NCO-Gruppe) oder -NR'-, worin R' H oder $C_1$ bis $C_4$-Alkyl ist (das heißt eine Aminogruppe oder eine substituierte Aminogruppe ist der Lieferant des aktiven H-Atoms), oder eine Einfachbindung, auch als direkte Bindung bezeichnet (das heißt eine COOH-Gruppe ist der Lieferant des aktiven H-Atoms, wobei als Ergebnis der Reaktion mit der NCO-Gruppe $CO_2$ gebildet wird und dadurch eine Einfachbindung resultiert).

s bedeutet vorzugsweise 1, 2 oder 3, mit der Maßgabe, daß im Falle von X = -O- oder -NR'- s = 1 ist, wobei R' H oder $C_1$ bis $C_4$ -Alkyl ist.

M bedeutet vorzugsweise H oder einen Basenrest, ausgewählt aus der Gruppe Alkalimetall, Ammonium und Organoammoniumion, wie K, Na, NH4, $H_2N(C_2H_5)_2$, $HN(CH_3)_3$, $H_3N(C_2H_4OH)$, $H_2N(C_2H_4OH)_2$, $HN(C_2H_4OH)_3$, $H_3N(C_{12}H_{25})$ und dergleichen.

Bezüglich der Bedeutungen von y und y' in Formel 1 ist es, wie bereits erwähnt, bevorzugt, daß (a) sowohl y als auch y' eine Zahl von 1 bis 7 ist, vorzugsweise eine Zahl von 1 bis 4, oder daß (b) entweder y oder y' 0 ist und das andere eine Zahl von 1 bis 7 ist, vorzugsweise 1 bis 4; die Variante (b) ist besonders bevorzugt. Es hat sich nämlich herausgestellt, daß die erfindungsgemäßen Urethane der Formel 1 dann besonders wirksam sind, wenn einer der beiden am Triisocyanat gebundenen Fluoralkoholstränge epichlorhydrinfrei ist, der andere aber Epichlorhydrin enthält, und zwar 1 bis 7, vorzugsweise 1 bis 4 Epichlorhydrin-Einheiten.

Die Herstellung der erfindungsgemäßen Urethane ergibt sich aus der Formel 1 und wird im folgenden näher beschrieben. Sie werden hergestellt durch Reaktion von 1 bis 3 mol von einem Fluoralkohol-Triisocyanat-Addukt der Formel 27

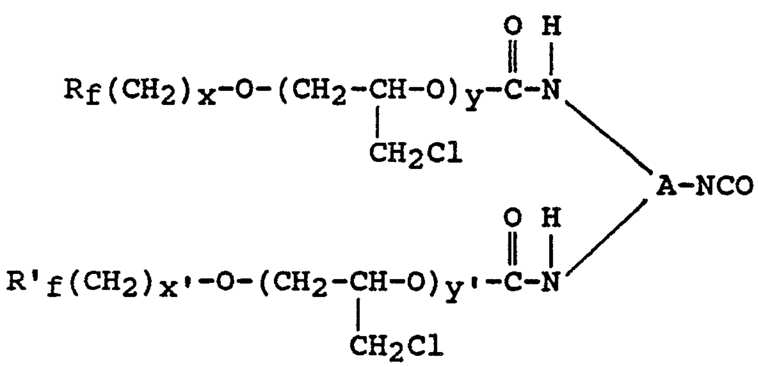

(27)

worin $R_f$, $R'_f$, x, x', y, y' und A die genannten Bedeutungen haben, mit 1 bis 3 mol von einer aromatischen, aliphatischen oder cycloaliphatischen Carbonsäure, wobei durch Addition von 1 bis 3 H-Atomen der Carbonsäureverbindung in Form von -OH, -NHR', worin R' die angegebene Bedeutung hat, oder von -COOH an die -NCO-Gruppe des Adduktes das angestrebte Urethan in Form einer Säure gebildet wird, aus dem man durch Neutralisation mit einer Base das angestrebte Urethan in Form eines Salzes erhält.

Die einzusetzenden Fluoralkohol-Triisocyanat-Addukte sind bekannt. Sie sind in der eingangs genannten US-Patentschrift 4 782 175 beschrieben. Ihre Herstellung erfolgt durch die Umsetzung des im Addukt enthaltenen aliphatischen Fluoralkohols mit einem Triisocyanat, entsprechend dem Rest A in Formel 27. Die Reaktion wird vorzugsweise bei Normaldruck und in Substanz oder unter Verwendung eines inerten Lösungsmittels durchgeführt. Geeignete Lösungsmittel sind halogenierte Kohlenwasserstoffe, wie Tetrachlorkohlenstoff (Siedepunkt 77 °C), Dichlorethan (Siedepunkt 84 °C) und Trifluortrichlorethan (Siedepunkt 48 °C), Ketone, wie Aceton (56 °C), Methylethylketon (80 °C) und Diethylketon (101 °C), Ether, wie Diisopropylether (68 °C), Tetrahydrofuran (66 °C) und Dibutylether (142 °C) und Ester, wie Ethylacetat (77 °C) und Butylacetat (123 °C). Insbesondere im Falle eines Lösungsmittels ist es bevorzugt, Lewis-Säuren als Katalysator einzusetzen. Die Art der Lewis-Säure ist nicht kritisch. Geeignete Lewis-Säuren sind $BF_3$, Bortrifluoriddiethyletherat, $SnCl_4$, $SbCl_5$, $TiCl_4$, $FeCl_3$, $PF_3$ und Dibutylzinndilaurat, wobei Bortrifluoriddiethyletherat und Dibutylzinndilaurat bevorzugt sind. Die Menge an Lewis-Säuren beträgt im allgemeinen 0,01 bis 3 Gew.-%, vorzugsweise 0,1 bis 1 Gew.-%, bezogen auf die Menge an eingesetztem Fluoralkohol. Die Reaktionstemperatur kann innerhalb weiter Grenzen variieren, sie liegt in der Regel im Bereich von 50 bis 150 °C. Es ist bevorzugt, die Reaktion bei der Siedepunkttemperatur des eingesetzten Lösungsmittels durchzuführen. Die Reaktionsdauer liegt bei 1 bis 5 Stunden. Die Reaktion verläuft quantitativ. Das erhaltene Fluoralkohol-Triisocyanat-Addukt ist im allgemeinen ein wachsartiges und farbloses oder leicht gelb bis braun gefärbtes Produkt.

Die Reaktion des Fluoralkohol-Triisocyanat-Adduktes mit der aliphatischen, aromatischen oder cycloaliphatischen Carbonsäure wird vorzugsweise derart durchgeführt, daß Addukt und Carbonsäure in einem Reaktionsgefäß vorgelegt werden und die Mischung unter Rühren bei einer Temperatur von 50 bis 150 °C, vorzugsweise 70 bis 120 °C, gehalten wird, bis keine freie NCO-Gruppe mehr vorhanden ist (der Verlauf der Reaktion kann zum Beispiel infrarotspektroskopisch verfolgt werden). Die Reaktion wird vorzugsweise bei Normaldruck und in Substanz oder unter Verwendung eines inerten Lösungsmittels durchgeführt. Die obengenannten Lösungsmittel sind auch hier geeignet. Die Gegenwart einer Lewis-Säure ist für den Verlauf der Reaktion vorteilhaft. Die obengenannten Lewis-Säuren sind auch hier geeignet. Die Menge an Lewis-Säure beträgt im allgemeinen 0,01 bis 3 Gew.-%, vorzugsweise 0,1 bis 1 Gew.-%, bezogen auf eingesetztes Addukt. Die Reaktionsdauer liegt im Bereich von 1 bis 8 Stunden. Die Reaktion verläuft quantitativ. Die erhaltenen Reaktionsprodukte sind die erfindungsgemäßen Urethane in Form der Säure, das heißt M = H. Sie stellen im allgemeinen ein festes bis wachsartiges, farbloses oder gelb bis braun gefärbtes Produkt dar. Das zur Umsetzung eingesetzte Lösungsmittel kann ohne Schwierigkeiten destillativ entfernt werden, wenn die Urethane in fester Form gewünscht werden.

Die Umwandlung der Urethane in Form der Säure in solche in Form von Salzen wird dadurch erreicht, daß man die erstgenannten mit einer Base, vorzugsweise mit einem Alkalimetallhydroxid, wie Natriumhydroxid oder Kaliumhydroxid, mit einem Alkalimetallalkoholat, wie Alkalimetallmethylat, -ethylat oder -isopropylat, oder mit Ammoniumhydroxid oder einer Organoammonium-Verbindung der obengenannten Art neutralisiert. Die Basen werden vorzugsweise in Form einer 10 bis 50 gew.%igen wäßrigen Lösung eingesetzt. Die erfindungsgemäßen Urethane in der Salzform liegen in der Regel als 20 bis 70 gew.%ige wäßrige Lösungen vor oder als Feststoff, das heißt im wesentlichen frei von Wasser oder anderen Lösungsmitteln.

Das aktive H-Atom bei der Reaktion der Carbonsäure mit dem Fluoralkohol-Triisocyanat-Addukt stammt, wie bereits erwähnt, von einer HO-Gruppe, HNR'-Gruppe oder COOH-Gruppe, die in den eingesetzten Mono- oder Polycarbonsäuren vorhanden sind. Es hat sich herausgestellt, daß die Bereitschaft, ein aktives H-Atom abzugeben, im allgemeinen in der folgenden Reihenfolge zunimmt: Carboxylgruppe, Hydroxylgruppe, Amino- oder substituierte Aminogruppe. Im Falle von COOH-Gruppen und HO-Gruppen in der eingesetzten Carbonsäure, was zum Beispiel bei Citronensäure zutrifft, ist also die HO-Gruppe der bevorzugte Lieferant des H-Atoms. Analoges gilt im Falle von COOH- und Amino- oder substituierten Aminogruppen.

Die neuen Urethane werden gemäß Erfindung zur Ausrüstung von Textilien verwendet. Sie verleihen den Textilien eine hervorragende Hydrophobie und Oleophobie. Sie weisen ferner die Eigenschaft auf, den starken Beanspruchungen, denen die ausgerüsteten Textilien ausgesetzt werden, beispielsweise beim Verstrecken, Texturieren und insbesondere beim Färben und Waschen, ohne nennenswerten Verlust an Wirkung standzuhalten. Ein weiterer Vorteil der erfindungsgemäßen Urethane liegt darin, daß sie auch in üblichen Textilbehandlungspräparationen, beispielsweise in Spinnpräparationen, eingesetzt werden können und dabei ihre hervorragende Wirkung nicht verlieren. Was die neuen Urethane besonders auszeichnet, ist die Feststellung, daß sie - zusätzlich zu den genannten Eigenschaften - auch den sogenannten Effekt der Trockenschmutz-Abweisung besitzen, das heißt die mit den erfindungsgemäßen Urethanen behandelten Textilien weisen auch festen Schmutz, wie Straßenstaub, Ruß und dergleichen, ab; auch dieser Schmutz wird vom behandelten Textil nicht angezogen und schon gar nicht festgehalten. Die erfindungsgemäßen Urethane verleihen also Textilmaterialien eine unerwartet vorteilhafte Eigenschaftskombination.

Das Textilmaterial kann natürlicher und/oder synthetischer Natur sein. Es besteht vorzugsweise aus Baumwolle, Polyamid, Polyester und/oder Polyacrylnitril, wobei Polyamid besonders bevorzugt ist. Das Textilmaterial kann in beliebiger Form vorliegen, so zum Beispiel als Faden, Faser, Garn, Gewebe, Teppich oder Vlies. Die Auftragsmenge an erfindungsgemäßer Verbindung wird so gewählt, daß auf dem Textilmaterial 0,05 bis 1,5 Gew.-% Fluor, vorzugsweise 0,1 bis 1 Gew.-% Fluor, vorhanden sind, Gewichtsprozente bezogen auf das behandelte Textilmaterial. Das Aufbringen der erfindungsgemäßen Urethane auf das Textilmaterial erfolgt in der Regel entweder während einer der üblichen Textilbehandlungen mit Präparationsmitteln, wobei das erfindungsgemäße Urethan dem Präparationsmittel einverleibt worden ist, oder mit Hilfe von Lösungen, Emulsionen oder Dispersionen, die bei der Herstellung der Urethane anfallen oder eigens bereitet worden sind. In den Textilbehandlungspräparationen, beispielsweise Spinnpräparationen, liegen die erfindungsgemäßen Urethane in einer Konzentration von 0,5 bis 5 Gew.-% vor, vorzugsweise 1 bis 3 Gew.-%. In den Lösungen, Emulsionen oder Dispersionen liegen sie in einer Konzentration von 5 bis 40 Gew.-% vor, vorzugsweise 8 bis 30 Gew.-%. Die Behandlung der Textilien mit den Lösungen, Emulsionen oder Dispersionen wird nach üblichen Methoden durchgeführt, so zum Beispiel durch Sprühen, Tauchen, Foulardieren und dergleichen. Anschließend wird das getränkte Textilmaterial getrocknet und einer Wärmebehandlung unterworfen. Die Wärmebehandlung (auch Kondensation genannt) wird in der

Regel in der Weise durchgeführt, daß das Textilmaterial auf eine Temperatur von 130 bis 200 °C erhitzt und bei dieser Temperatur 10 Sekunden bis 10 Minuten lang gehalten wird. Das mit den erfindungsgemäßen Urethanen ausgerüstete Textilmaterial besitzt die oben erwähnten hervorragenden Eigenschaften.

Die Erfindung wird nun anhand von Beispielen noch näher erläutert.

Erfindungsgemäße Verbindungen

In den Beispielen zur Herstellung der erfindungsgemäßen Urethane wurden die folgenden Fluoralkohol-Triisocyanat-Addukte eingesetzt:

**Addukt 1:**

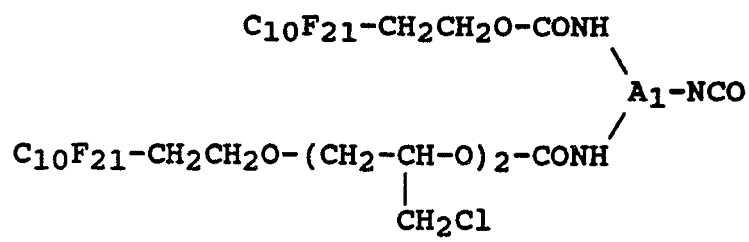

**Addukt 2:**

$$(C_{10}F_{21}-CH_2CH_2O-CONH)_2-A_1-NCO$$

**Addukt 3:**

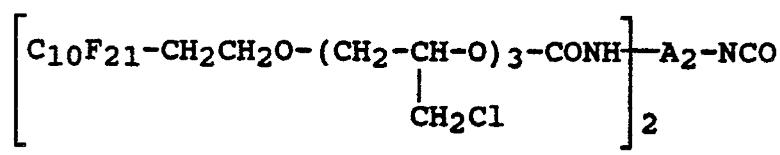

**Addukt 4:**

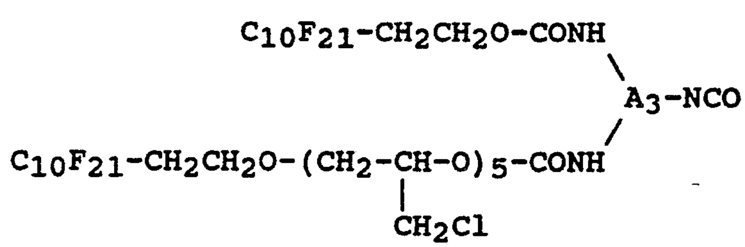

A1 bedeutet einen Triisocyanatrest entsprechend dem Triisocyanat der Formel 2.

$A_2$ bedeutet einen Triisocyanatrest entsprechend dem Triisocyanat der Formel 6.

$A_3$ bedeutet einen Triisocyanatrest entsprechend dem Triisocyanat der Formel 7.

Der Perfluoralkylrest $C_{10}F_{21}$- im Fluoralkohol der Addukte 1 bis 4 steht für ein ($C_8F_{17}$-$C_{16}F_{33}$)-Gemisch, das kurz mit $C_{10}F_{21}$- bezeichnet wird.

Nachdem Fluoralkohol-Triisocyanat-Addukte und ihre Herstellung zum Stand der Technik gehören und eine bevorzugte Herstellungsart oben bereits beschrieben worden ist, dürfte sich ein weiteres Eingehen auf die angegebenen und in den folgenden Beispielen eingesetzten Addukte 1 bis 4 erübrigen.

Beispiel 1

85,0 g (0,021 mol) vom Addukt 1 und 4,1 g (0,021 mol) Citronensäure, pulverförmig, sowie 30 g Methylethylketon als Lösungsmittel (Dispergiermittel) und 3 Tropfen Dibutylzinndilaurat als Katalysator wurden in einem Reaktionsgefäß vorgelegt, das mit einem Rührer, Thermometer und einem Rückflußkühler mit Trockenröhrchen ausgestattet war. Die Mischung wurde auf 80 °C erhitzt (Siedetemperatur von Methylethylketon) und bei dieser Tempertur 4 Stunden lang unter Rühren gehalten, wobei Addukt 1 und Citronensäure zur angestrebten erfindungsgemäßen Urethanverbindung reagierten. Das mit einer Ausbeute von 95,8 % der Theorie erhaltene Urethan (ein wachsartiges, gelbgefärbtes Produkt) ist in der nachstehenden Tabelle 1 formelmäßig angegeben; Formel B1.

Beispiel 2

Durchführung wie in Beispiel 1, wobei 278,1 g (0,070 mol) vom Addukt 1 und 5,3 g (0,070 mol) Glykolsäure ($HOCH_2COOH$) eingesetzt wurden. Die Ausbeute an Urethanverbindung betrug 94,4 % der Theorie; Formel B2.

Beispiel 3

Durchführung wie in Beispiel 1, wobei 85,0 g (0,021 mol) vom Addukt 1 und 3,8 g (0,021 mol) von der nachstehenden aromatischen Aminodicarbonsäure

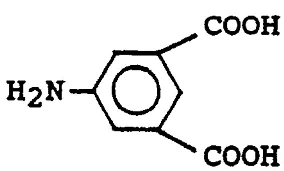

eingesetzt wurden. Die aus Addukt 1 und der Aminodicarbonsäure gebildete Urethanverbindung wurde in das entsprechende Natriumsalz übergeführt. Dazu wurde das nach der vierstündigen Umsetzung von Addukt 1 und Aminodicarbonsäure erhaltene Reaktionsprodukt mit der erforderlichen Menge an Natriummethylat (das sind 7,6 g) in Form einer 30gew.%igen methanolischen Lösung versetzt und die Mischung bei einer Temperatur im Bereich von 40 bis 60 °C 30 Minuten lang unter Rühren gehalten. Die Ausbeute an Urethanverbindung in Form eines Natriumsalzes (ein wachsartiges, gelbgefärbtes Produkt) betrug 97,1 % der Theorie; Formel B3.

Beispiel 4

Durchfürung wie in Beispiel 3, wobei 85,0 g (0,021 mol) vom Addukt 1 und 4,3 g (0,021 mol) Aminoundecansäure eingesetzt wurden und die Mischung bei den genannten 80 °C 8 Stunden lang unter Rühren gehalten wurde. Die Ausbeute an Urethanverbindung in Form eines Natriumsalzes betrug 96,6 % der Theorie; Formel B4.

Beispiel 5

Durchführung wie in Beispiel 4, wobei 150,0 g (0,048 mol) vom Addukt 1 und 6,3 g (0,048 mol) Aminocapronsäure eingesetzt wurden. Die Ausbeute an Urethanverbindung in Form eines Natriumsalzes betrug 98,4 % der Theorie; Formel B5.

Beispiel 6

Durchführung wie in Beispiel 1, wobei 270,6 g (0,069 mol) vom Addukt 2 und 7,3 g (0,023 mol) von der nachstehenden Benzoltetracarbonsäure

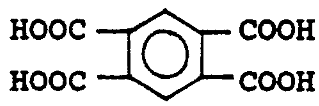

eingesetzt wurden. Die Ausbeute an Urethanverbindung betrug 97,9 % der Theorie; Formel B6.

Beispiel 7

Durchführung wie in Beispiel 1, wobei 245,0 g (0,063 mol) vom Addukt 1 und 9,2 g (0,063 mol) Adipinsäure eingesetzt wurden und die Mischung bei den genannten 80 °C 10 Stunden lang unter Rühren gehalten wurde. Die Ausbeute an Urethanverbindung betrug 96,9 % der Theorie; Formel B7.

Beispiel 8

Durchführung wie in Beispiel 2. Die aus Addukt 1 und der Glykolsäure gebildete Urethanverbindung wurde mit Monoethanolamin in das entsprechende Salz übergeführt. Dazu wurde das nach der Umsetzung von Addukt 1 und Glykolsäure erhaltene Reaktionsprodukt mit der erforderlichen Menge an Monoethanolamin ($H_2NCH_2CH_2OH$) versetzt und die Mischung bei einer Temperatur im Bereich von 40 bis 60 °C 30 Minuten lang unter Rühren gehalten. Die Ausbeute an Urethanverbindung in Form eines Ethanolaminsalzes (ein mehr oder weniger pulverförmiges, gelbgefärbtes Produkt) betrug 98,0 % der Theorie; Formel B8.

Beispiel 9

Durchführung wie in Beispiel 8, wobei die Salzbildung mit Dodecylmonoamin ($H_2NC_{12}H_{25}$) durchgeführt wurde. Die Ausbeute an Urethanverbindung in Form eines Dodecylaminsalzes (ein mehr oder weniger pulverförmiges, gelbgefärbtes Produkt) betrug 98,0 % der Theorie; Formel B9.

Beispiel 10

Durchführung wie in Beispiel 6, wobei 50,5 g (0,0094 mol) vom Addukt 2 und 1,5 g (0,0047 mol) Benzoltetracarbonsäure eingesetzt wurden. Die Ausbeute an Urethanverbindung betrug 97,5 % der Theorie; Formel B10.

Beispiel 11

Durchführung wie in Beispiel 1, wobei 102,0 g (0,024 mol) vom Addukt 3 und 4,6 g (0,024 mol) Äpfelsäure eingesetzt wurden. Die Ausbeute an Urethanverbindung betrug 97,5 % der Theorie; Formel B11.

Beispiel 12

Durchführung wie in Beispiel 1, wobei 90,0 g (0,019 mol) vom Addukt 4 und 3,7 g (0,019 mol) Citronensäure eingesetzt wurden. Die Ausbeute an Urethanverbindung betrug 97,8 % der Theorie; Formel B12.

TABELLE 1

| Nr. | Formeln der erfindungsgemäßen Urethane nach den Beispielen 1 bis 12 |
| --- | --- |
| B1 | 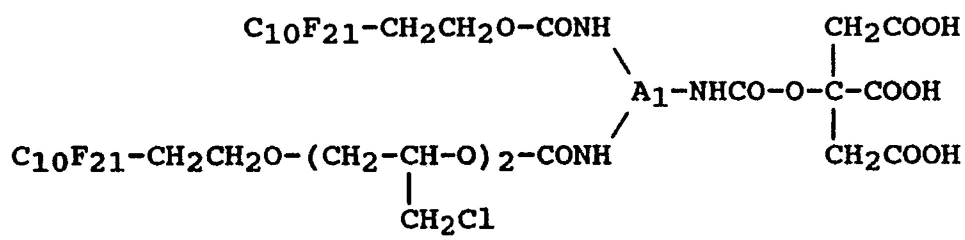 |
| B2 | 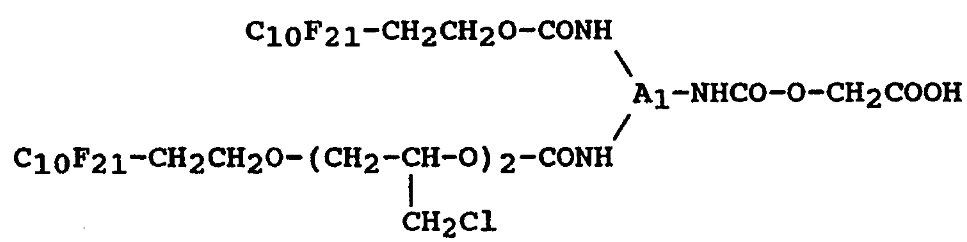 |
| B3 | 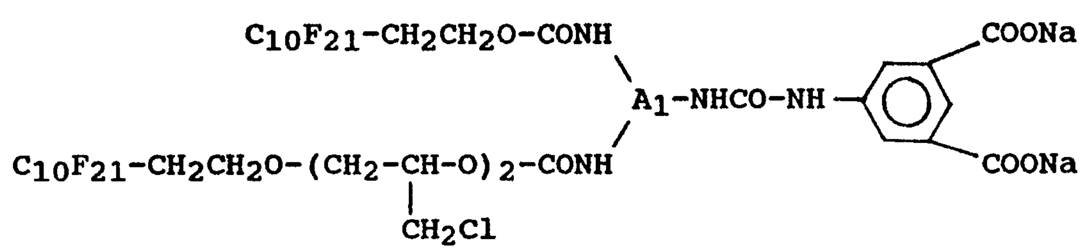 |

(Fortsetzung)

T A B E L L E 1 (Fortsetzung)

| Nr. | Formeln der erfindungsgemäßen Urethane nach den Beispielen 1 bis 12 |
|---|---|
| B4 | 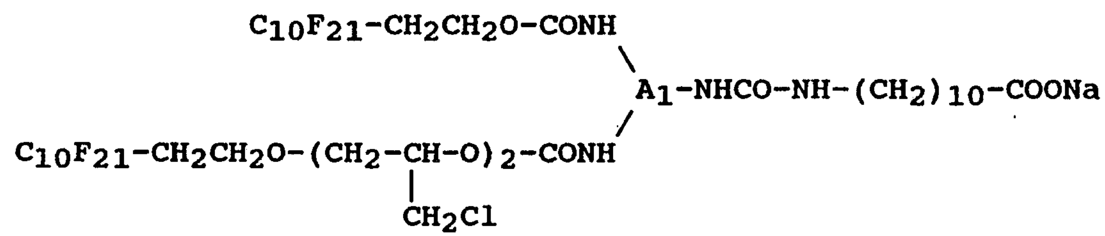 |
| B5 | 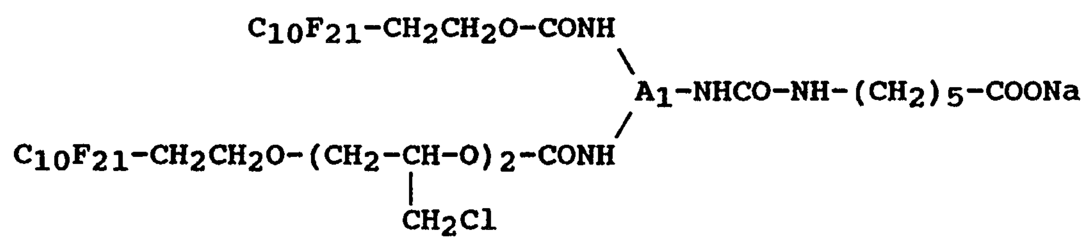 |
| B6 | 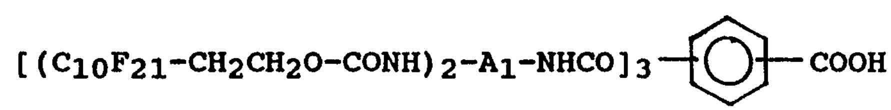 |

(Fortsetzung)

T A B E L L E 1 (Fortsetzung)

| Nr. | Formeln der erfindungsgemäßen Urethane nach den Beispielen 1 bis 12 |
|---|---|
| B7 | 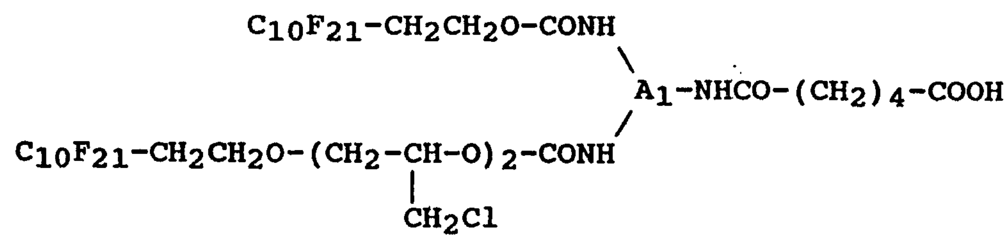 |
| B8 | 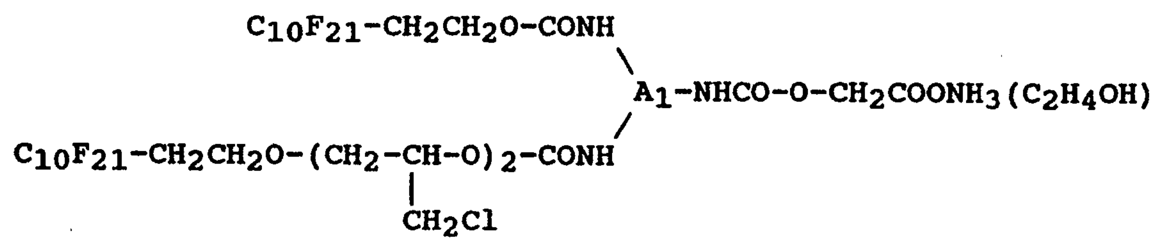 |
| B9 | 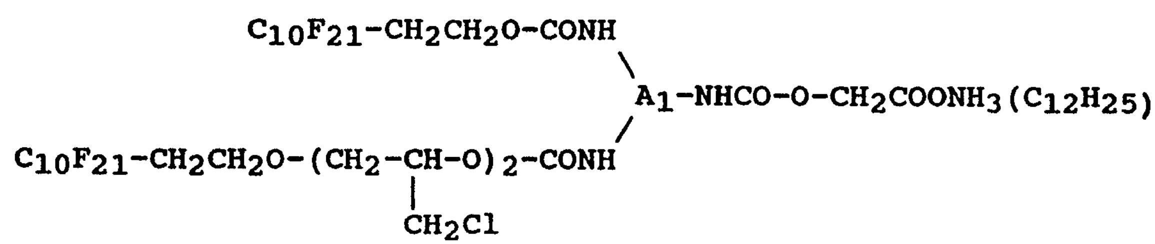 |

(Fortsetzung)

TABELLE 1 (Fortsetzung)

| Nr. | Formeln der erfindungsgemäßen Urethane nach den Beispielen 1 bis 12 |
|---|---|
| B10 | 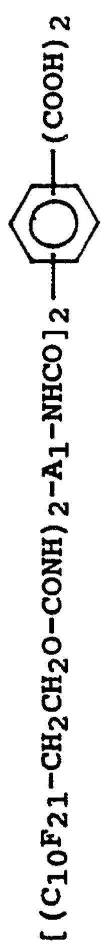 |
| B11 | 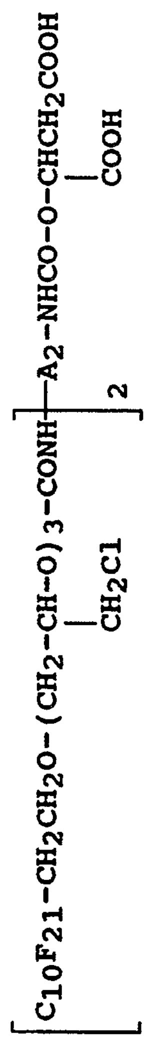 |
| B12 | 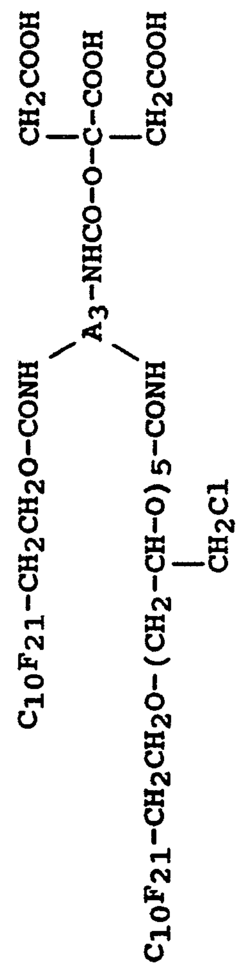 |

Verwendung der erfindungsgemäßen Verbindungen

Beispiele I bis XII

In den Beispielen I bis XII wurden die erfindungsgemäßen Verbindungen B1 bis B12 mit Hilfe einer üblichen Spinnpräparation für Polyamidfasern getestet, die jeweils circa 150 g erfindungsgemäße Verbindung pro 1 000 g Spinnpräparation enthielt (die Spinnpräparation bestand also aus Wasser als Hauptkomponente, den üblichen ethoxylierten Fettalkoholen und langkettigen Aminoxiden als Präparationsmittel und circa 15 Gew.-% erfindungsgemäßer Verbindung). Mit jeder der zwölf Spinnpräparationen wurden jeweils gleiche Polyamid-6-Filamente behandelt, um soviel von der erfindungsgemäßen Verbindung und dem Präparationsmittel auf die Filamente aufzubringen, daß 0,05 Gew.-% Fluor und 1 Gew.-% Präparationsmittel auf den Filamenten vorlagen, Gewichtsprozente jeweils bezogen auf das Gewicht der Filamente. Dazu wurden die Filamente in üblicher Weise durch die Spinnpräparation gezogen, getrocknet und 30 Sekunden lang bei einer Temperatur von 200 °C gehalten (Wärmebehandlung, Kondensation). Aus den so behandelten Filamenten wurde jeweils ein Gewebe hergestellt. Es lagen zwölf Gewebe mit den erfindungsgemäßen Verbindungen B1 bis B12 vor, wobei auf jedem Gewebe eine Fluorauflage von 0,05 Gew.-% und eine Präparationsmittelauflage von 1 Gew.-% vorhanden waren, Gewichtsprozente jeweils bezogen auf das Gewicht des Gewebes.

An den zwölf Geweben wurden die Ölabweisung (Oleophobie) nach der AATCC-Prüfnorm 118 - 1966, die Wasserabweisung (Hydrophobie) nach DIN 53 888 - 1965 und die Anschmutzung (Trockenschmutz-Abweisung) nach der weiter unten beschriebenen Methode geprüft, und zwar nach der erwähnten einer dreistündigen Behandlung der kondensierten Gewebe mit einer alkalischen Kochwäsche. Bei dieser Behandlung wurden die einzelnen Gewebe in üblicher Weise 3 Stunden lang in einer alkalischen Waschflüssigkeit gekocht und anschließend getrocknet; die Waschflüssigkeit bestand aus 1 1 Wasser, 1 g Trinatriumphosphat und 2 g eines Fettsäurepolyglykolesters, der durch Oxethylierung von Butandiol-1,4 mit 15 mol Ethylenoxid und anschließender Veresterung des Oxethylats mit 1 mol Ölsäure erhalten worden ist.

Die Ergebnisse aus den Beispielen I bis XII sind in der nachstehenden Tabelle 2 zusammengefaßt.

TABELLE 2

| Beispiele und getestete Verbindungen | Ölabweisung | | Wasserabweisung | | Anschmutzung | |
|---|---|---|---|---|---|---|
| | nach der Kondensation | nach der Kochwäsche | nach der Kondensation | nach der Kochwäsche | nach der Kondensation | nach der Kochwäsche |
| I/B1 | 6 | 5 | 5 | 5 | 3 | 4 |
| II/B2 | 6 | 5 | 5 | 5 | 3 | 4 |
| III/B3 | 6 | 5 | 5 | 5 | 3 | 4 |
| IV/B4 | 6 | 6 | 5 | 5 | 3 | 3 |
| V/B5 | 6 | 5 | 5 | 5 | 3 | 4 |
| VI/B6 | 5 | 4 | 5 | 4 | 3 | 3 |
| VII/B7 | 6 | 5 | 6 | 5 | 3 | 3 |
| VIII/B8 | 6 | 5 | 5 | 4 | 3 | 4 |
| IX/B9 | 6 | 5 | 5 | 4 | 3 | 4 |
| X/B10 | 5 | 4 | 5 | 4 | 3 | 3 |
| XI/B11 | 5 | 4 | 5 | 4 | 3 | 4 |
| XII/B12 | 6 | 5 | 6 | 5 | 3 | 4 |

Im folgenden werden der AATCC-Test 118 - 1966 (American Association of Textile Chemists and Colorists), DIN 53 888 - 1965 (Deutsche Industrie-Norm) und die Bestimmung der Naßanschmutzung beschrieben.

Zur Bestimmung des Ölabweisungswertes nach AATCC-Test 118 - 1966 werden bekanntlich 3 Tropfen

von einer bestimmten Testflüssigkeit (siehe unten) vorsichtig auf das zu prüfende Textilmaterial aufgelegt. Einwirkungszeit: 30 Sekunden. Es wird der Wert angegeben, bei dem noch keine augenscheinliche Benetzung des Gewebes unter den Tropfen (nach abgelaufener Einwirkungszeit) hervorgerufen worden ist:

| Testflüssigkeit | Ölabweisungswert |
|---|---|
| Paraffinöl | 1 |
| Paraffinöl : n-Hexadecan = 65 : 35 | 2 |
| n-Hexadecan | 3 |
| n-Tetradecan | 4 |
| n-Dodecan | 5 |
| n-Decan | 6 |
| n-Octan | 7 |
| n-Hepan | 8 |

Ein ölabweisungswert von 1 bedeutet den schlechtesten und ein ölabweisungswert von 8 den besten Abweisungseffekt.

Zur Bestimmung des Wasserabweisungswertes nach DIN 53 888 - 1965 werden bekanntlich die zu prüfenden Textilien unter standardisierten Bedingungen beregnet, wobei gleichzeitig die Unterseite der Textilprobe mechanisch gerieben wird. Es wird der Wasserabperleffekt visuell mit den Noten 1 bis 5 beurteilt, wobei Note 1 den schlechtesten und Note 5 den besten Abperleffekt bedeutet.

Die Bestimmung der Anschmutzung wurde nach der folgenden Methode durchgeführt: Das zu prüfende Textilmaterial wird in einer Drehtrommel mit einer Schmutzflotte bei 60 °C 45 Minuten lang behandelt. Die Schmutzflotte besteht aus 2 g von einem üblichen Vollwaschmittel und 1 g Schmutz in 1 l Wasser. Der Schmutz ist eine Mischung aus Straßenstaub mit einer Partikelgröße von maximal 0,4 µm als Hauptkomponente und 3 Gew.-% (bezogen auf Straßenstaub) von einem Rußmehl. Die während der genannten 45 Minuten in Rotation befindliche Drehtrommel enthält auf 1 Gew.-Teil des zu prüfenden Textilmaterials 50 Gew.-Teile Schmutzflotte. Das so behandelte Textilmaterial wird mit Wasser gespült und in einem Trockenschrank bei 50 °C getrocknet. Das angeschmutzte und getrocknete Textilmaterial wird nun einer Wäsche unterzogen. Dazu wird es in einer Drehtrommel mit einer Reinflotte bei 60 °C 30 Minuten lang behandelt. Die Reinflotte besteht aus 2 g des in der ersten Behandlung verwendeten Vollwaschmittels in 1 l Wasser. Die während der genannten 30 Minuten in Rotation befindliche Drehtrommel enthält auf 1 Gew.-Teil des zur Waschung eingesetzten Textilmaterials 50 Gew.-Teile Reinflotte. Das so gewaschene Textilmaterial wird mit Wasser gespült und in einem Trockenschrank bei 50 °C getrocknet. An dem nun vorliegenden Textilmaterial wird der Graumaßstab visuell und mit den Ncten 1 bis 5 beurteilt; die Note 1 stellt das schlechteste Ergebnis dar (das heißt das Textilmaterial hat durch noch anhaftenden Schmutz graues Aussehen), während die Note 5 das beste Ergebnis ist (das heißt das Textilmaterial ist vollständig frei von Schmutz).

Die Testergebnisse zeigen, daß mit den erfindungsgemäßen Urethanen neben einer hohen öl- und Wasserabweisung auch eine überraschend hohe Schmutzabweisung erreicht wird.

## Ansprüche

1. Urethane aus aliphatischen Fluoralkoholen, Isocyanaten und Carbonsäuren der nachstehenden Formel 1

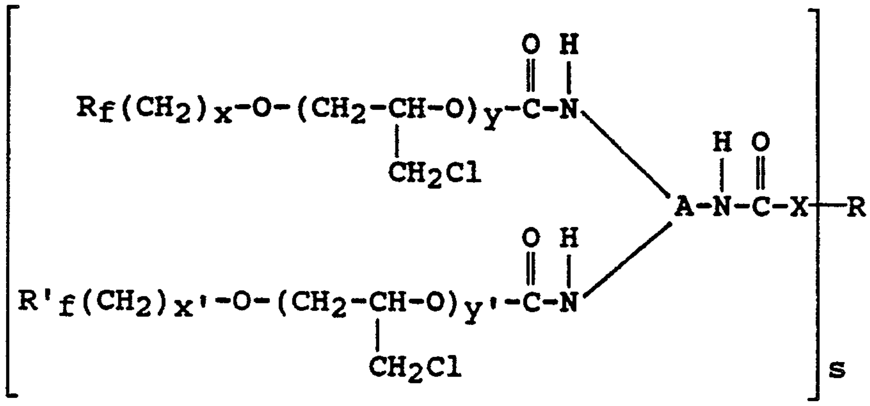 (1)

worin bedeuten:
$R_f$ und $R'_f$ jeweils eine Perfluoralkylgruppe mit 4 bis 22 C-Atomen,
x und x' jeweils eine Zahl von 1 bis 4,
y und y' jeweils eine Zahl von 0 bis 7,
A einen Rest eines aliphatischen, aromatischen oder cycloaliphatischen Triisocyanates,
R einen Rest einer aromatischen, aliphatischen oder cycloaliphatischen Carbonsäure oder Carbonsäuresalzes, der 1 bis 5 -COOM-Gruppen aufweist, worin M Wasserstoff oder ein Basenrest ist,
X -O-, eine Einfachbindung oder -NR'-, worin R' H, $C_1$ bis $C_4$-Alkyl oder $-CH_2COOM$ ist, worin M die genannte Bedeutung hat, und
s eine Zahl von 1 bis 3,
mit der Maßgabe, daß diejenigen Urethanverbindungen ausgenommen sind, wenn $R_f$ und $R'_f$ jeweils $(C_8F_{17}-C_{16}F_{33})$-, x und x' und y und y' jeweils 2 sind, A ein Triisocyanatrest der nachstehenden Formel ist,

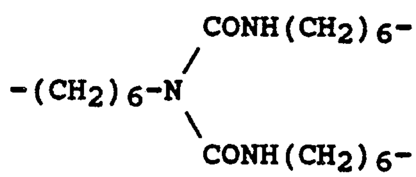

X -O- oder -NH- ist, R ein aromatischer Carbonsäurerest der nachstehenden Formel

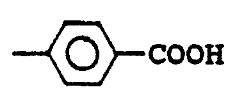

und s 1 ist.

2. Urethane nach Anspruch 1, wobei bedeuten:
$R_f$ und $R'_f$ jeweils eine Perfluoralkylgruppe mit 6 bis 18 C-Atomen,
x und x' jeweils 2,
y und y' jeweils eine Zahl von 1 bis 7 oder einer der beiden Indices 0 und der andere eine Zahl von 1 bis 7,
A einen Rest eines aliphatischen oder aromatischen Triisocyanates,
R einen Rest einer aromatischen Carbonsäure oder eines aromatischen Carbonsäuresalzes der nachstehenden Formeln 8 und 9:

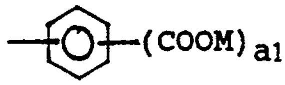

(8)

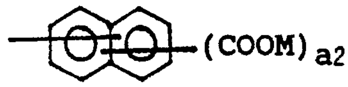

(9)

worin M die angegebene Bedeutung hat und a1 und a2 jeweils eine Zahl von 1 bis 5 sind, oder einen Rest einer aliphatischen Carbonsäure oder eines aliphatischen Carbonsäuresalzes der nachstehenden Formel 10

$$-R^1-COOM \qquad (10)$$

worin M die angegebene Bedeutung hat und $R^1$ ein aliphatischer Kohlenwasserstoffrest mit maximal 15 C-Atomen ist,
X -O-, eine Einfachbindung oder -NR'-, worin R' H oder $C_1$ bis $C_4$-Alkyl ist, und
s eine Zahl von 1 bis 3, mit der Maßgabe, daß im Falle von X = -O- oder -NR'- s = 1 ist.

3. Urethane nach Anspruch 1, wobei bedeuten:
$R_f$ und $R'_f$ jeweils eine Perfluoralkylgruppe mit 6 bis 18 C-Atomen,
x und x' jeweils 2,
y und y' jeweils eine Zahl von 1 bis 7 oder einer der beiden Indices 0 und der andere eine Zahl von 1 bis 7,
A einen Rest eines aliphatischen oder aromatischen Triisocyanates,
R einen Rest einer aromatischen Carbonsäure oder eines aromatischen Carbonsäuresalzes der nachstehenden Formeln 8 und 9:

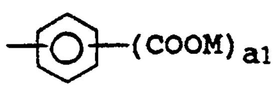

(8)

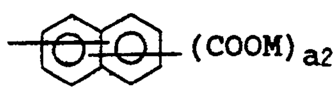

(9)

worin M die angegebene Bedeutung hat und a1 und a2 1, 2 oder 3 sind, oder einen Rest einer aliphatischen Carbonsäure oder eines aliphatischen Carbonsäuresalzes der nachstehenden Formel 10

$$-R^1-COOM \qquad (10)$$

worin H die angegebene Bedeutung hat und $R^1$ ein $-(CH_2)_z$-Rest ist, z = 1 bis 10, oder ein Rest der nachstehenden Formeln 11 bis 13 ist,

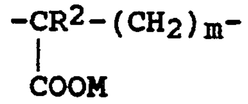 (11)

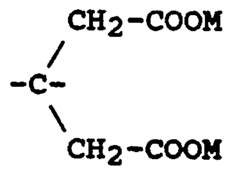 (12)

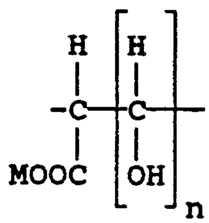 (13)

worin M die angegebene Bedeutung hat, m 0 oder eine Zahl von 1 bis 7 ist, n 1, 2 oder 3 ist und $R^2$ H, OH oder $C_1$ bis $C_4$-Alkyl ist,
X -O-, eine Einfachbindung oder -NR'-, worin R' H oder $C_1$ bis $C_4$-Alkyl ist, und
s eine Zahl von 1 bis 3, mit der Maßgabe, daß im Falle von x = -O- oder -NR'- s = 1 ist.

**4.** Verfahren zur Herstellung der Urethane nach Anspruch 1, gekennzeichnet durch die Reaktion von 1 bis 3 mol von einem Fluoralkohol-Triisocyanat-Addukt der Formel 27

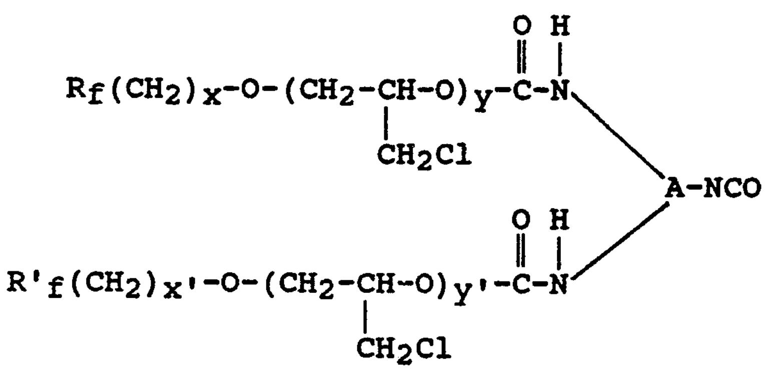 (27)

worin $R_f$, $R'_f$, x, x', y, y' und A die genannten Bedeutungen haben, mit 1 bis 3 mol von einer aromatischen, aliphatischen oder cycloaliphatischen Carbonsäure, wobei durch Addition von 1 bis 3 H-Atomen der Carbonsäureverbindung in Form von -OH, -NHR', worin R' die angegebene Bedeutung hat, oder von -COOH an die -NCO-Gruppe des Adduktes das angestrebte Urethan in Form einer Säure gebildet wird, aus dem man durch Neutralisation mit einer Base das angestrebte Urethan in Form eines Salzes erhält.

**5.** Verwendung der Urethane nach Anspruch 1 zur Ausrüstung von Textilien in der Wasser-, öl- und Schmutzabweisung.